# DEMANDE DE BREVET EUROPEEN

(11) **EP 2 319 483 A1**
(43) Date de publication de la demande: **11.05.2011**
(21) Numéro de dépôt: 10177530.2
(22) Date de dépôt: 20.09.2010
(51) Int. Cl.: A61K 8/06, A61Q 1/02

(54) **Ensemble de conditionnement et d'application cosmétique pour compositions rhéofluidifiantes**

(30) Priorité: 21.09.2009 FR 0956483
(71) Demandeur: L'Oréal, 75008 Paris (FR)
(72) Inventeur: Arnaud, Pascal, 94240, L'Hay Les Roses (FR); Vidal, Marie, 81100, Castres (FR)
(74) Mandataire: Leonard, Armelle

(57) **Abrégé**

La présente invention concerne un ensemble de conditionnement et d'application cosmétique de maquillage et/ou de soin des matières kératiniques comprenant au moins :
- un récipient contenant au moins une composition cosmétique, sous forme d'émulsion eau-dans-huile caractérisée par le profil rhéologique suivant :
- une viscosité comprise entre 35 et 1000 Pa s, en particulier entre 40 et 950 Pa s, de préférence entre 55 et 900 Pa s, à un gradient de cisaillement de 0,01 s⁻¹,
- une viscosité comprise entre 0,20 et 0,90 Pa s, en particulier entre 0,25 et 0,80 Pa s, de préférence entre 0,28 et 0,70 Pa s, à un gradient de cisaillement de 1000 s⁻¹,
telles que mesurées à l'aide d'un rhéomètre à contrainte imposée, ARG2 TA instrument, à 25°C, et
- une tête applicatrice (3) solidaire dudit récipient et supportant un organe d'application (7), ledit organe d'application comportant une surface d'application apte, en réponse à son déplacement en engagement avec les matières kératiniques, à tourner autour d'au moins un axe ou d'un centre de rotation, ladite surface d'application étant en communication fluidique avec ledit récipient via au moins un passage qui, sur au moins une partie de sa longueur, présente un diamètre de 1 à 6mm, de préférence de 2 à 5mm, et mieux de 2 à 4mm.

## Description

La présente invention vise à proposer un nouveau mode de maquillage et/ou de soin des matières kératiniques et plus particulièrement dédié à l'application sur une matière kératinique, en particulier sur la peau, de compositions cosmétiques fluides sous forme d'émulsions eau-dans-huile.

Par matières kératiniques selon l'invention, on entend notamment la peau et les lèvres, en particulier la peau, et notamment la peau du visage.

Les compositions de fond de teint sont couramment employées pour apporter une couleur esthétique à la peau, notamment au visage, mais également pour camoufler les imperfections de la peau, telles que les rougeurs et/ou les taches. Plus particulièrement, dans le domaine des compositions de maquillage et/ou de soin de texture fluide, il est courant d'utiliser des compositions sous forme d'émulsions eau-dans-huile (E/H), comportant une phase aqueuse dispersée dans une phase huileuse.

Les consommatrices recherchent de plus en plus des produits faciles et rapides à appliquer, qui ne salissent pas les mains et dont le dépôt sur la peau, est le plus homogène possible.
La praticité de l'application est d'autant plus recherchée que le produit est liquide, car dans ce cas, sa préhension et son application sur la peau, sont plus délicates. En effet, le produit peut être alors difficile à doser, couler entre les doigts et tacher les vêtements.
Dans le cas d'un produit de maquillage, comme les fonds de teint fluides, les consommatrices sont obligées de se laver les mains après l'application, ce qui est une perte de temps et parfois ne peut pas être réalisé dans de bonnes conditions en particulier dans les transports.

L'homogénéité est aussi un critère important qui n'est pas toujours obtenue lors de l'application du produit avec les doigts. Or cette homogénéité du dépôt est fondamentale tant sur le plan esthétique, pour un produit de maquillage, que sur le plan de l'efficacité pour un produit solaire ou un produit de soin.
Afin de facilité l'application, il est connu de l'homme de l'art d'utiliser des applicateurs comme des pinceaux ou des sprays. Mais ceux-ci ne permettent pas d'obtenir une homogénéité de dépôt optimale.
Les poils du pinceau peuvent laisser des trainées au cours de l'application et les sprays conduisent à un dépôt dont l'épaisseur peut varier beaucoup d'un endroit à l'autre du visage ou du corps en fonction de la gestuelle d'application.

Il reste donc nécessaire de rechercher une solution technique permettant d'obtenir à la fois une bonne praticité et un dépôt homogène à l'application.

La présente invention vise précisément à répondre à ce besoin.

Plus particulièrement, la présente invention concerne, selon un de ses aspects, un ensemble cosmétique de conditionnement et d'application cosmétique de maquillage et/ou de soin des matières kératiniques, comprenant au moins :
- un récipient contenant au moins une composition cosmétique, sous forme d'émulsion eau-dans-huile caractérisée par le profil rhéologique suivant :
- une viscosité comprise entre 35 et 1000 Pa s, en particulier entre 40 et 950 Pa s, de préférence entre 55 et 900 Pa s, à un gradient de cisaillement de 0,01 s⁻¹,
- une viscosité comprise entre 0,20 et 0,90 Pa s, en particulier entre 0,25 et 0,80 Pa s, de préférence entre 0,28 et 0,70 Pa s, à un gradient de cisaillement de 1000 s⁻¹, telles que mesurées à l'aide d'un rhéomètre à contrainte imposée, ARG2 TA instrument, à 25°C, et
   - une tête applicatrice (3) solidaire dudit récipient et supportant un organe d'application (7), ledit organe d'application comportant une surface d'application apte, en réponse à son déplacement en engagement avec les matières kératiniques, à tourner autour d'au moins un axe ou d'un centre de rotation, ladite surface d'application étant en communication fluidique avec ledit récipient via au moins un passage qui, sur au moins une partie de sa longueur, présente un diamètre de 1 à 6mm, de préférence de 2 à 5mm, et mieux de 2 à 4mm.

Selon un mode de réalisation préféré, ledit passage débouche en regard de ladite surface d'application via au moins un orifice présentant un diamètre de 1 à 6mm, de préférence de 2 à 5mm, et mieux de 2 à 4mm.

Par diamètre, on entend notamment le diamètre inscrit dans la forme géométrique de l'orifice, pour le cas où l'orifice aurait une forme non-circulaire.
Selon un mode préféré, l'orifice a une forme circulaire.

Les inventeurs ont en effet observé que :
■ A faible cisaillement (de 10⁻³ à 10⁻¹ s⁻¹), une corrélation peut être faite entre la viscosité de la composition et la capacité à amorcer l'applicateur : plus la viscosité est faible plus l'amorçage est facile.
■ A fort cisaillement (1000 s⁻¹), une corrélation peut être faite entre la viscosité de la composition et la quantité de composition restituée : la viscosité ne doit être ni trop élevée ni trop basse. Par ailleurs, une corrélation qualitative peut être réalisée entre la viscosité de la composition et la capacité à appliquer cette composition via l'applicateur.

La composition selon l'invention peut être une composition de maquillage et/ou de soin de la peau en particulier du visage et/ou du corps, et peut constituer un fard à joues, un fard à paupières, un fond de teint, un produit anticerne, un rouge à lèvres, un produit de maquillage du corps, un produit de soin du visage ou du corps ou un produit antisolaire.

Plus spécialement mais non exclusivement, l'invention porte sur une composition de fond de teint.

L'émulsion selon l'invention possède une caractéristique rhéologique spécifique qui rend son utilisation particulièrement intéressante. Ainsi, lors de l'application sur la matière kératinique, notamment la peau, sous la pression exercée par l'applicateur selon l'invention, l'émulsion se fluidifie sous l'effet du cisaillement dû au déplacement de l'organe d'application sur la peau.
On dit que la composition selon l'invention a un caractère ou un comportement rhéofluidifiant, c'est-à-dire que la viscosité de la composition peut être abaissée, de manière réversible, lorsque l'on applique à la composition des cisaillements croissants. Une telle composition présente des niveaux de viscosité différents à faible (10⁻² s⁻¹) et fort cisaillement (10³ s⁻¹).

### Mesures de Viscosité

Les mesures de viscosité sont effectuées à l'aide d'un rhéomètre à contrainte imposée, ARG2 TA instrument, équipé d'un corps de mesure cône-plan muni d'un dispositif anti-évaporation (cloche). Les mesures sont réalisées à 25°C.
Une mesure de la viscosité est réalisée à toutes les vitesses de cisaillement comprises entre 0,001 et 1000 s⁻¹ en se conformant au protocole rhéologique suivant :
■ *Précisaillement* : on effectue un cisaillement à 1000 s⁻¹ pendant 1 min suivi d'un temps de repos de 10 min.
■ *Cycle de cisaillement en vitesse contrôlée :* on réalise une rampe ascendante logarithmique de 0,001 à 1000 s⁻¹ en 2 min suivie d'un cisaillement à 1000 s⁻¹ pendant 1 min et enfin une rampe descendante logarithmique de 1000 à 0,001 s⁻¹ en 2 min.

Selon un autre aspect, l'invention concerne un procédé de maquillage et/ou de soin d'une matière kératinique, en particulier la peau, dans lequel on prélève et on applique simultanément une quantité d'une composition, notamment sous forme de fond de teint fluide, à l'aide d'un dispositif d'application selon l'invention, comprenant un récipient contenant ladite composition et une tête applicatrice solidaire dudit récipient supportant un organe d'application, ledit organe d'application comportant une surface d'application apte, en réponse à son déplacement en engagement avec les matières kératiniques, à tourner autour d'un axe ou d'un centre de rotation, notamment tel que défini ci-après, ladite composition étant sous forme d'émulsion eau-dans-huile caractérisée par le profil rhéologique suivant :
- une viscosité comprise entre 35 et 1000 Pa s, en particulier entre 40 et 950 Pa s, de préférence entre 55 et 900 Pa s, à un gradient de cisaillement de 0,01 s⁻¹,
- une viscosité comprise entre 0,20 et 0,90 Pa s, en particulier entre 0,25 et 0,80 Pa s, de préférence entre 0,28 et 0,70 Pa s, à un gradient de cisaillement de 1000 s⁻¹,
telles que mesurées à l'aide d'un rhéomètre à contrainte imposée, ARG2 TA instrument, à 25°C, ladite surface d'application étant en communication fluidique avec ledit récipient via au moins un passage qui, sur au moins une partie de sa longueur, présente un diamètre de 1 à 6mm, de préférence de 2 à 5mm, et mieux de 2 à 4mm.

Selon un mode particulier, le dispositif d'application est **caractérisé en ce que** le passage débouche sur ladite surface d'application via au moins un orifice présentant un diamètre de 1 à 6mm, de préférence de 2 à 5mm, et mieux de 2 à 4mm.

### DISPOSITIF D'APPLICATION

Le dispositif d'application selon l'invention comprend un récipient et une tête applicatrice supportant un organe d'application, l'organe d'application comportant une surface d'application apte, en réponse à son déplacement en engagement avec les matières kératiniques, en particulier la peau, à tourner autour d'au moins un axe ou centre de rotation.

On parle de dispositifs d'application de type « distributeur-applicateur » tels que ceux décrits dans la demande WO2006/090061.

Lors de l'application, l'organe d'application peut tourner sans glisser sur la peau. Plusieurs passages successifs peuvent être effectués à un même endroit, selon par exemple l'intensité de couleur recherchée. Lors des passages successifs, l'utilisateur peut modifier légèrement la direction de roulement, afin d'estomper les bords du dépôt de produit.

Un tel dispositif d'application permet une bonne prise en main et est très maniable.

En outre, la mise en oeuvre d'un organe d'application conforme à l'invention pour effectuer le dépôt d'une composition en surface d'une matière kératinique et plus particulièrement de la peau permet d'obtenir un effet massant sur la peau, de nature à procurer des sensations sensorielles agréables pour l'utilisateur.

Le mode d'application selon l'invention présente par ailleurs l'avantage de ne pas se salir les doigts avec la composition à l'application.

Par ailleurs, la mise en oeuvre d'un organe d'application selon l'invention permet d'obtenir un maquillage significativement amélioré en termes d'homogénéité de dépôt, procurant un effet de maquillage discret et naturel.

L'organe d'application peut être défini par un rouleau, une bille ou encore une bande disposée autour de deux rouleaux possédant des axes de rotation parallèles. De préférence, il s'agit d'un rouleau, donc distinct d'une bille.

Selon un mode préféré, ladite surface d'application est sous la forme d'une mousse, différent de matériaux plastiques ou métalliques généralement utilisés pour les systèmes rotatifs bille ou 'roll-on', tels que les applicateurs 'roll-on' déodorants et anti-transpirants.

En particulier, l'utilisation d'un rouleau présentant une large surface d'application en contact avec les matières kératiniques et en particulier la peau, améliore la qualité du résultat maquillage, notamment en terme d'homogénéité du dépôt.

L'organe d'application se présente avantageusement sous la forme d'un rouleau, creux ou non, qui tourne autour d'un axe de rotation. Cet axe de rotation peut être avantageusement disposé perpendiculairement à l'axe longitudinal du dispositif.

La tête applicatrice qui porte l'organe d'application peut être mobile relativement au récipient, notamment rotative par rapport à celui-ci, notamment autour de son axe longitudinal. Le déplacement de la tête applicatrice relativement au récipient peut provoquer celui d'un obturateur, l'amenant à passer d'une position d'obturation fermant une communication entre le ou les orifices et le récipient à une position ouverte, et inversement (position on/off).
La tête applicatrice peut comporter deux montants entre lesquels tourne l'organe d'application.
La tête applicatrice et/ou le récipient peut également comporter un ou plusieurs canaux permettant d'amener le produit à la surface de l'organe d'application.

La surface d'application dédiée à un contact avec une matière kératinique peut notamment être une mousse à cellules ouvertes ou fermées, éventuellement floquées, un flocage, un élastomère, un matériau fritté, un matériau tissé ou un matériau non-tissé. De préférence, il s'agit d'une mousse à cellules ouvertes ou fermées.

La surface d'application peut être lisse ou non. Ainsi, cet organe d'application peut avantageusement posséder en surface, des reliefs, généralement bombés et arrondis avantageux pour procurer conjointement un effet de massage.

Dans le dispositif utilisé selon l'invention, la surface d'application est en communication fluidique avec ledit récipient via au moins un passage qui, sur au moins une partie de sa longueur, présente un diamètre de 1 à 6mm, de préférence de 2 à 5mm, et mieux de 2 à 4mm.
Selon un mode de réalisation préféré, le passage débouche en regard de ladite surface d'application via au moins un orifice présentant un diamètre de 1 à 6mm, de préférence de 2 à 5mm, et mieux de 2 à 4mm.

La composition stockée dans le récipient est ainsi directement délivrée à la surface de l'organe d'application depuis l'extérieur, par au moins un orifice présentant un diamètre de 1 à 6mm, de préférence de 2 à 5mm et mieux de 2 à 4mm. L'imprégnation de l'organe d'application avec la composition, et son déplacement sur la matière kératinique sont donc réalisés simultanément.

Le récipient peut comporter un volume variable pour mettre le produit sous pression et forcer sa distribution à travers le ou les orifices.
Selon un premier mode de réalisation, le récipient peut comporter une paroi souple, l'utilisateur pouvant appuyer sur cette paroi et mettre ainsi le produit sous pression. Selon une variante de réalisation, le récipient comprend un piston que l'utilisateur peut actionner pour mettre le produit sous pression et forcer sa distribution à travers le ou les orifices.

L'orifice peut communiquer de façon sélective avec le récipient : le passage peut comprendre une valve et/ou un système obturateur.

L'obturateur peut être mobile relativement au récipient entre une position d'obturation où il ferme la communication entre l'orifice et le récipient, et une position de distribution où il rétablit ladite communication.

### Exemples d'ensembles de conditionnement et d'application cosmétiques pour la mise en oeuvre de l'invention

On a représenté sur les figures un exemple parmi d'autres de dispositif d'application de type 'distributeur-applicateur' pouvant être utilisé selon l'invention, et particulièrement adapté à l'application d'une composition fluide présentant le profil rhéologique selon l'invention.

Sur le dessin :
- la figure 1 représente, de manière schématique, en perspective, un exemple d'ensemble de conditionnement et d'application conforme à l'invention,
- la figure 2 représente, en coupe longitudinale dans l'axe Y, partielle et schématique, l'exemple d'ensemble de conditionnement et d'application de la figure 1 conforme à l'invention,
- la figure 3 représente, en coupe longitudinale dans l'axe Y, partielle et schématique, une variante d'ensemble de conditionnement et d'application conforme à l'invention (avec piston).

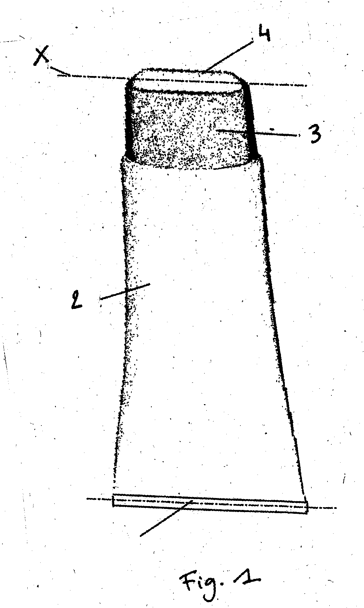

Selon un mode de réalisation, le dispositif de conditionnement et d'application 1, représenté en Figure 1, se présente sous la forme d'un tube distributeur-applicateur, adapté au stockage, distribution et application d'un produit P fluide, plus ou moins visqueux.

De tels applicateurs-distributeurs sont notamment décrits dans la demande WO 2006/090061.

Un tel dispositif peut comporter un récipient de stockage de la composition sous forme d'un tube souple 2, comportant à son extrémité une tête applicatrice 3, supportant un organe d'application 4.

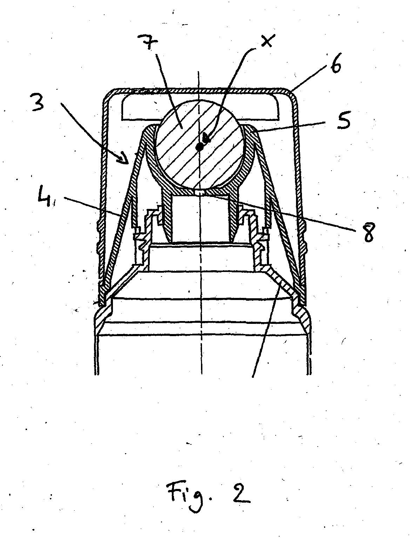

Comme représenté à la Figure 2, la tête applicatrice 3 est montée sur le col du tube via une jupe d'accrochage 4. La tête comporte en outre un support creux 5 portant l'organe d'application. La tête est montée sur le tube de façon étanche grâce à une jupe d'étanchéité.

Le tube souple 2 peut prendre toute forme appropriée, et être de différent matériau, notamment plastique ou thermoplastique.

Avantageusement, la tête est surmontée d'un capuchon adapté 6 qui protège le dispositif d'application au cours du stockage du tube.

L'organe d'application 7 peut se présenter sous la forme d'un rouleau, dont la surface d'application peut être cylindrique de révolution autour de l'axe X. Cet organe d'application est apte à tourner et peut prélever d'un côté, le produit provenant du récipient, tout en l'appliquant de l'autre côté.

Le rouleau applicateur 7 peut tourner ainsi dans ledit support creux, alimenté en produit via un orifice 8 présentant un diamètre de 3mm, et entraîner le produit jusqu'à la matière kératinique.

Pour utiliser le dispositif de conditionnement et d'application représenté en figures 1 et 2, l'utilisateur peut, afin d'alimenter la tête applicatrice 3 en produit, appuyer sur la paroi souple du tube 2, tout en continuant à appliquer ledit organe d'application 7 sur la matière kératinique à traiter, par exemple la peau.

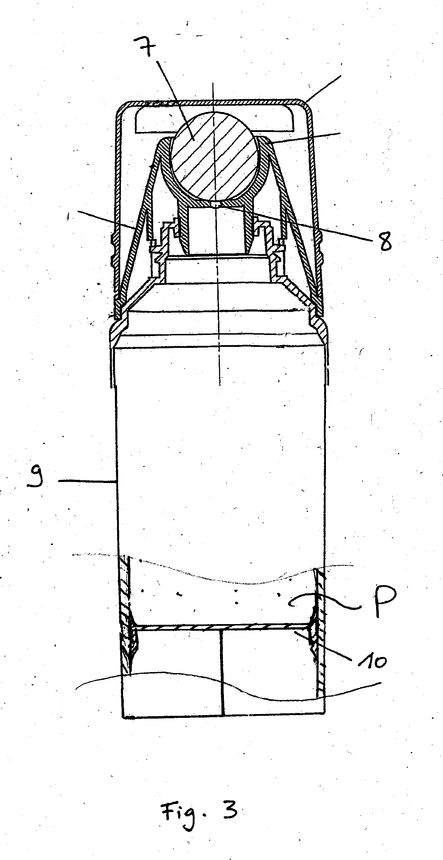

Selon une autre variante de réalisation, l'ensemble de conditionnement et d'application, représenté en figure 3, peut comporter un récipient 9 de stockage du produit P, dans lequel peut coulisser un piston 10, permettant de forcer le produit P, vers l'organe d'application 7, alimenté par au moins un canal intérieur 8.

### COMPOSITION

La composition selon l'invention est sous la forme d'une émulsion eau-dans-huile, comprenant une phase aqueuse dispersée dans une phase grasse liquide, et comprend au moins un milieu physiologiquement acceptable.

Par « milieu physiologiquement acceptable », on entend désigner un milieu convenant particulièrement à l'application d'une composition de l'invention sur les matières kératiniques, notamment la peau et les lèvres.

Le milieu physiologiquement acceptable est généralement adapté à la nature du support sur lequel doit être appliquée la composition, ainsi qu'à l'aspect sous lequel la composition doit être conditionnée.

Ladite composition est caractérisée par le profil rhéologique défini précédemment, à savoir :
- une viscosité comprise entre 35 et 1000 Pa s, en particulier entre 40 et 950 Pa s, de préférence entre 55 et 900 Pa s, à un gradient de cisaillement de 0,01 s⁻¹,
- une viscosité comprise entre 0,20 et 0,90 Pa s, en particulier entre 0,25 et 0,80 Pa s, de préférence entre 0,28 et 0,70 Pa s, à un gradient de cisaillement de 1000 s⁻¹, telles que mesurées à l'aide d'un rhéomètre à contrainte imposée, ARG2 TA instrument, à 25°C.

Elle est caractérisée notamment par une phase aqueuse dispersée dans une phase grasse liquide, un tensioactif et la présence éventuellement d'au moins un agent destiné à augmenter la viscosité de ladite composition.

### PHASE AQUEUSE

Une composition selon l'invention comprend au moins une phase aqueuse, de préférence en une teneur allant de 10 à 80 %, et plus particulièrement de 30 à 70 % en poids par rapport au poids total de la composition.

La phase aqueuse comprend de l'eau et/ou au moins un solvant hydrosoluble.

L'eau peut être une eau florale telle que l'eau de bleuet et/ou une eau minérale telle que l'eau de VITTEL, l'eau de LUCAS ou l'eau de LA ROCHE POSAY et/ou une eau thermale.

Par « solvant hydrosoluble », on désigne dans la présente invention un composé liquide à température ambiante et miscible à l'eau (miscibilité dans l'eau supérieure à 50 % en poids à 25 °C et pression atmosphérique).

Le ou les solvant(s) hydrosolubles convenant à l'invention peu(vent)t être choisi(s) parmi les monoalcools en C₁₋₈, et notamment en C₁₋₅, notamment l'éthanol, l'isopropanol, le tert-butanol, le n-butanol, les polyols tels que décrits précédemment, et leurs mélanges. Conviennent aussi tout particulièrement à l'invention, l'éthanol et l'isopropanol et de préférence l'éthanol.

Une composition de l'invention peut en outre comprendre au moins un sel, par exemple, le chlorure de sodium, le chlorure de magnésium et le sulfate de magnésium.

Une composition de l'invention peut comprendre de 0,05 % à 1,5 % en particulier de 0,1 % à 1,0 % et plus particulièrement de 0,15 % à 0,8 % en poids de sels, par rapport au poids total de la composition.

La phase aqueuse peut également comprendre tout composé hydrosoluble ou hydrodispersible compatible avec une phase aqueuse tels que des gélifiants, des polymères filmogènes, des épaississants, des tensio-actifs et leurs mélanges.

### PHASE GRASSE LIQUIDE

Une composition cosmétique conforme à la présente invention comprend au moins une phase grasse liquide et notamment au moins une huile comme mentionnée ci-après.

On entend par huile, tout corps gras sous forme liquide à température ambiante (20 - 25 °C) et à pression atmosphérique.

Une composition de l'invention peut comprendre une phase grasse liquide en une teneur variant de 10 à 90 %, en particulier de 15 à 60 %, et plus particulièrement, de 20 à 50 % en poids par rapport au poids total de la composition.

Une composition selon l'invention peut comprendre au moins une huile, choisie parmi les huiles volatiles et non volatiles de type hydrocarbonées, siliconées, ou fluorées. Les huiles peuvent être d'origine animale, végétale, minérale ou synthétique.

On entend par huile, tout corps gras sous forme liquide à température ambiante (20-25 °C) et à pression atmosphérique.

Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg).

En outre, l'huile volatile a généralement un point d'ébullition, mesuré à pression atmosphérique, allant de 150 °C à 260 °C, et de préférence allant de 170 °C à 250 °C.

Avantageusement, la phase grasse comprend de 5 % à 40 % en poids, de préférence allant de 10 % à 35 % en poids, et préférentiellement allant de 15 % à 30 % en poids d'huile(s) volatile(s) par rapport au poids total de la composition.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor ; elle peut contenir des groupes ester, éther, amine, amide.

Par huile siliconée, on entend une huile contenant au moins un atome de silicium, et notamment contenant des groupes Si-O.

Par huile fluorée, on entend une huile contenant au moins un atome de fluor.

### Huiles volatiles

L'huile volatile hydrocarbonée utilisable dans l'invention peut être choisie parmi les huiles hydrocarbonées ayant un point éclair allant de 40 °C à 102 °C, de préférence allant de 40 °C à 55 °C, et préférentiellement allant de 40 °C à 50 °C.

Comme huile volatile hydrocarbonée, on peut citer les huiles volatiles hydrocarbonées ayant de 8 à 16 atomes de carbone et leurs mélanges, et notamment les alcanes ramifiés en C₈-C₁₆ comme les iso-alcanes (appelées aussi isoparaffines) en C₈-C₁₆, l'isododécane, l'isodécane, l'isohexadécane et par exemple les huiles vendues sous les noms commerciaux d'Isopars ou de Permetyls, les esters ramifiés en C₈-C₁₆ comme le néopentanoate d'iso-hexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isododécane.

L'huile volatile hydrocarbonée peut être présente en une teneur allant de 5 % à 20 % en poids, par rapport au poids total de l'émulsion, de préférence allant de 6 % à 15 % en poids, et préférentiellement allant de 7 % à 12 % en poids.

L'huile volatile siliconée utilisable dans l'invention peut être choisie parmi les huiles siliconées ayant un point éclair allant de 40 °C à 102 °C, de préférence ayant un point éclair supérieur à 55 °C et inférieur ou égal à 95 °C, et préférentiellement allant de 65 °C à 95 °C.

Comme huile volatile siliconée, on peut citer les huiles de silicones linéaires ou cycliques ayant de 2 à 7 atomes de silicium, ces silicones comportant éventuellement des groupes alkyle ou alkoxy ayant de 1 à 10 atomes de carbone. Comme exemple d'huile volatile siliconée, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

Comme huile volatile fluorée, on peut citer le nonafluoroéthoxybutane, le nonafluorométhoxybutane, le décafluoropentane, le tétradécafluorohexane, le dodécafluoropentane, et leurs mélanges.

L'huile volatile choisie parmi les huiles volatiles siliconées, les huiles volatiles fluorées, et leurs mélanges peut être présente en une teneur allant de 0 % à 32 % en poids, par rapport au poids total de l'émulsion, de préférence allant de 10 % à 30 % en poids, et préférentiellement allant de 15 % à 25 % en poids par rapport au poids total de la composition.

La phase grasse de l'émulsion selon l'invention peut comprendre en outre au moins une huile non volatile.

### Huiles non volatiles

Cette huile non volatile ou un de ses mélanges peut être présente en une teneur allant de 0,1 % à 40 % en poids, par rapport au poids total de l'émulsion, et de préférence allant de 1 % à 25 % en poids.

L'huile non volatile peut être choisie parmi les huiles d'origine minérale, animale, végétale ou synthétique, carbonées, hydrocarbonées et/ou siliconées, et leurs mélanges, dans la mesure où elles sont compatibles avec l'utilisation envisagée.

On peut citer les huiles hydrocarbonées non volatiles telles que l'huile de paraffine ou de vaseline, l'isoeicosane, de soja, le perhydrosqualène, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol.

Comme huile non volatile siliconée, on peut citer les polydiméthylsiloxane (PDMS), éventuellement phénylées telles que les phényltriméthicones, ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, et leurs mélanges.

### AGENTS TENSIOACTIFS

Une composition selon l'invention comprend un ou plusieurs agent(s) tensioactif(s) adaptés aux émulsions E/H, notamment choisi(s) parmi les agents tensioactifs amphotères, anioniques, cationiques ou non ioniques, utilisés seuls ou en mélange.

Les agents tensioactifs sont généralement présents dans la composition, en une teneur pouvant aller de 0,5 à 15 % en poids, en particulier de 1,5 à 5% en poids, par rapport au poids total de la composition.

A titre d'exemple, le ou les tensioactif(s) siliconé(s) peut/peuvent être présent(s) en une teneur allant de 0,5 à 10 % en poids, notamment de 1 à 5 % en poids, par rapport au poids total de la composition.

Selon un autre mode de réalisation particulier, le ou les tensioactif(s) non siliconé(s) peut/peuvent être présent(s) en une teneur allant de 1 à 10 % en poids, notamment de 2 à 8 % en poids, par rapport au poids total de la composition.

Pour les émulsions E/H, on peut utiliser notamment des agents tensioactifs hydrocarbonés ou siliconés.

On peut citer par exemple comme agents tensioactifs hydrocarbonés, les polyesters de polyols comme le PEG-30 dipolyhydroxystearate vendu sous la référence ARLACEL P 135 par la société Uniqema, le polyglyceryl-2 dipolyhydroxystearate vendu sous la référence DEHYMULS PGPH par la société Cognis.

On peut citer par exemple comme agents tensioactifs siliconés les alkyl-diméthicone copolyols tels que le Laurylmethicone copolyol vendu sous la dénomination "Dow Corning 5200 Formulation Aid" par la société Dow Corning et le Cetyl dimethicone copolyol vendu sous la dénomination ABIL EM 90 par la société Goldschmidt, ou le mélange polyglycéryl-4 isostéarate/cétyl diméthicone copolyol/hexyllaurate vendu sous la dénomination ABIL WE 09 par la société Goldschmidt.

On peut y ajouter aussi un ou plusieurs co-émulsionnants. De manière avantageuse, le co-émulsionnant peut être choisi dans le groupe comprenant les esters alkylés de polyol. Comme esters alkylés de polyol, on peut citer notamment les esters de glycérol et/ou de sorbitan et par exemple le polyglyceryl-3 diisostéarate commercialisé sous la dénomination de LAMEFORM TGI par la société Cognis, l'isostéarate de polyglycérol-4, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Selon un mode de réalisation particulièrement préféré, une émulsion selon l'invention, en particulier une émulsion E/H comprenant une phase huileuse siliconée, comprend au moins un tensioactif siliconé, et plus particulièrement choisi parmi les diméthicone copolyols.

La présence d'un diméthicone copolyol favorise notamment la stabilisation de l'émulsion selon l'invention.

Un diméthicone copolyol utilisable selon l'invention est un polydiméthyl méthyl siloxane oxypropyléné et/ou oxyéthyléné.

On peut utiliser comme diméthicone copolyol ceux répondant à la formule (II) suivante : 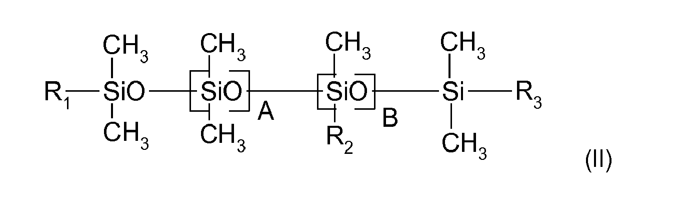 dans laquelle :
- R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle en C₁-C₃ ou un radical acyle en C₂-C₄ ;
- A est un nombre entier allant de 0 à 200 ;
- B est un nombre entier allant de 0 à 50 ; à la condition que A et B ne soient pas égaux à zéro en même temps ;
- x est un nombre entier allant de 1 à 6 ;
- y est un nombre entier allant de 1 à 30 ; et
- z est un nombre entier allant de 0 à 5.

Selon un mode de réalisation préféré de l'invention, dans le composé de formule (II), R₁ = R₃ = radical méthyle, x est un nombre entier allant de 2 à 6 et y est un nombre entier allant de 4 à 30. R₄ est en particulier un hydrogène.

On peut citer, à titre d'exemples de composés de formule (II), les composés de formule (III) : 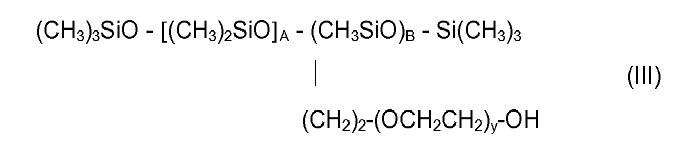 dans laquelle A est un nombre entier allant de 20 à 105, B est un nombre entier allant de 2 à 10 et y est un nombre entier allant de 10 à 20.

On peut également citer à titre d'exemple de composés siliconés de formule (II), les composés de formule (IV) :

HO - (OCH₂CH₂)_{y}-(CH₂)₃ - 1(CH₃)₂SiO]_{A'} - (CH₂)₃ - (OCH₂CH₂)_{y} - OH (IV)

dans laquelle A' et y sont des nombres entiers allant de 10 à 20.

On peut utiliser comme diméthicone copolyols ceux vendus sous les dénominations DC 5329, DC 7439-146, DC 2-5695, Q4-3667 par la société Dow Corning ; KF-6013, KF-6015, KF-6016, KF-6017, KF-6028 par la société Shin-Etsu.

Les composés DC 5329, DC 7439-146, DC 2-5695 sont des composés de formule (III) où respectivement A est 22, B est 2 et y est 12 ; A est 103, B est 10 et y est 12 ; A est 27, B est 3 et y est 12.

Selon un mode de réalisation particulier, le tensioactif siliconé peut être le PEG-polydiméthylsiloxyéthyldiméthicone, notamment commercialisé par la société Shin-Etsu sous la référence KF-6028, le PEG-1 0 diméthicone notamment commercialisé par la société Shin-Etsu sous la référence KF-6017, et leurs mélanges.

Le diméthicone copolyol peut être présent dans la composition selon l'invention en une teneur allant de 1 % à 6 % en poids, par rapport au poids total de l'émulsion, et de préférence allant de 1,5 % à 4 % en poids, et préférentiellement allant de 2 % à 3 % en poids.

Selon une variante de réalisation préférée de l'invention, le diméthicone copolyol précité peut être associé à au moins une silicone oxyalkylénée substituée en α-ω.

### Silicone oxyalkylénée substituée en α-ω

Dans tout ce qui suit ou qui précède, on entend désigner par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₁₀ et en particulier méthyle, les radicaux fluoroalkyles, les radicaux aryles et en particulier phényle. Ils peuvent être par exemple substitués par des groupements ester ou éther en C₁-C₄₀, des groupements aralkyles en C₇-C₆₀.

Ainsi, la silicone oxyalkyléne substituée en α-ω utilisable selon l'invention est un polymère organosilicié tel que défini ci-dessus, à structure linéaire, substitué aux deux extrémités de la chaîne principale par des groupements oxyalkylène reliés aux atomes de Si par l'intermédiaire d'un groupement hydrocarboné.

De préférence, la silicone oxyalkylénée substituée en α-ω répond à la formule générale (I) suivante : 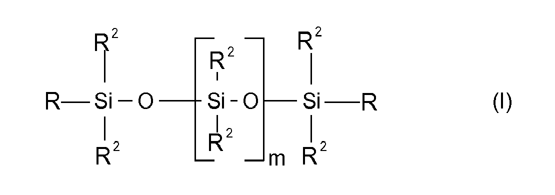 dans laquelle : R = -(CH₂)ₚ O-(C₂H₄O)ₓ (C₃H₆O)_{Y}R¹ où :
- R¹ représente H, CH₃ ou CH₂CH₃,
- p est un entier allant de 1 à 5, x varie de 1 à 100, y varie de 0 à 50,
- les unités (C₂H₄O) et (C₃H₆O) pouvant être réparties de façon aléatoire ou par blocs,
- les radicaux R² représentent un radical alkyle en C₁-C₃ ou un radical phényle, et
- 5≤m≤300.

De préférence, la silicone oxyalkylénée substituée en α-ω utilisée selon la présente invention répond à la formule générale (I) pour laquelle tous les radicaux R² sont des radicaux méthyles et :
- p va de 2 à 4,
- x va de 3 à 100,
- m va de 50 à 200.

De préférence encore, le poids moléculaire moyen de R va de 800 à 2600.

De préférence, le rapport en poids des unités C₂H₄O par rapport aux unités C₃H₆O va de 100:10 à 20:80. De façon avantageuse, ce rapport est d'environ 42/58.

De préférence encore, R¹ est le groupe méthyle.

De façon plus préférentielle encore, l'émulsion selon l'invention comprend la silicone oxyalkylénée en α-ω de formule suivante : 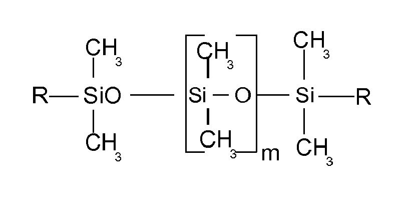 dans laquelle :
- m = 100,
- R = (CH₂)₃-O-(C₂H₄O)ₓ-(C₃H₆O)_{y}-CH₃, où x va de 3 à 100, y va de 1 à 50, le rapport en poids du nombre de C₂H₄O sur le nombre de C₃H₆O étant d'environ 42/58, le poids moléculaire moyen de R allant de 800 à 1000.

La silicone oxyalkylénée substituée en α-ω telle que définie ci-dessus peut être utilisée selon l'invention en une proportion allant de 0,5 à 5 % en particulier de 1 à 4 % en poids et plus particulièrement de 2 à 3 % en poids par rapport au poids total de la composition.

Parmi les produits du commerce pouvant contenir tout ou partie des silicones oxyalkylénées substituées en α-ω utilisables selon l'invention comme émulsionnant, on peut citer notamment ceux vendus sous les dénominations de "Abil EM 97" par la Société Goldschmidt, ou encore de "KF 6009", "X22-4350", "X22-4349" ou "KF 6008" par la Société Shin Etsu.

En particulier, il peut s'agir du cétyl diméthicone copolyol.

### Elastomères de silicone émulsionnants

On peut également utiliser comme agents tensioactifs d'émulsions E/H, un élastomère de silicone émulsionnant.

Avantageusement, une composition selon l'invention comprend au moins un élastomère de silicone émulsionnant, éventuellement en association avec un dimethicone copolyol tel que décrit précédemment.

L'élastomère de silicone émulsionnant peut être choisi parmi un élastomère de silicone polyoxylakyléné, un élastomère de silicone polyglycérolé, et leurs mélanges.

De préférence, on utilisera un élastomère de silicone polyglycérolé.

L'élastomère de silicone polyoxyalkyléné est un organopolysiloxane réticulé pouvant être obtenu par réaction d'addition réticulation de diorganopolysiloxane contenant au moins un hydrogène lié au silicium et d'un polyoxyalkylène ayant au moins deux groupements à insaturation éthylénique.

De préférence, l'organopolysiloxane réticulé polyoxyalkyléné est obtenu par réaction d'addition réticulation (A1) de diorganopolysiloxane contenant au moins deux hydrogènes liés chacun à un silicium, et (B1) de polyoxyalkylène ayant au moins deux groupements à insaturation éthylénique, notamment en présence (C1) de catalyseur platine, comme par exemple décrit dans les brevets US 5,236,986 et US 5,412,004.

En particulier, l'organopolysiloxane peut être obtenu par réaction de polyoxyalkylène (notamment polyoxyéthylène et/ou polyoxypropylène) à terminaisons diméthylvinylsiloxy et de méthylhydrogénopolysiloxane à terminaisons triméthylsiloxy, en présence de catalyseur platine.

Les groupes organiques liés aux atomes de silicium du composé (A1) peuvent être des groupes alkyles ayant de 1 à 18 atomes de carbone, tels que méthyle, éthyle, propyle, butyle, octyle, décyle, dodécyle (ou lauryle), myristyle, cétyle, stéaryle ; des groupes alkyles substitués tels que 2-phényléthyle, 2-phénylpropyle, 3,3,3-trifluoropropyle ; des groupes aryles tels que phényle, tolyle, xylyle ; des groupes aryles substitués tels que phényléthyle ; et des groupes hydrocarbonés monovalents substitués tels qu'un groupe époxy, un groupe ester carboxylate, ou un groupe mercapto.

Le composé (A1) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (C1) est le catalyseur de la réaction de réticulation, et est notamment l'acide chloroplatinique, les complexes acide chloroplatinique-oléfine, les complexes acide chloroplatinique-alkénylsiloxane, les complexes acide chloroplatinique-dicétone, le platine noir, et le platine sur support.

Avantageusement, les élastomères de silicone polyoxyalkylénés peuvent être formés à partir de composés divinyliques, en particulier des polyoxyalkylènes ayant au moins deux groupes vinyliques, réagissant avec des liaisons Si-H d'un polysiloxane.

L'élastomère de silicone polyoxyalkyléné selon l'invention est de préférence mélangé avec au moins une huile hydrocarbonée et/ou une huile siliconée pour former un gel. Dans ces gels, l'élastomère polyoxyalkyléné est sous forme de particules non-sphériques.

Des élastomères polyoxyalkylénés sont notamment décrits dans les brevets US 5,236,986, US 5,412,004, US 5,837,793, US 5,811,487.

Comme élastomère de silicone polyoxyalkyléné, on peut utiliser ceux commercialisés sous les dénominations "KSG-21", "KSG-20", "KSG-30", "KSG-31", KSG-32", "KSG-33", "KSG-210", "KSG-310", "KSG-320", "KSG-330", "KSG-340", "X-226146" par la société Shin Etsu, "DC9010", "DC9011" par la société Dow Corning.

Selon un autre mode de réalisation, l'élastomère de silicone émulsionnant peut être avantageusement choisi parmi les élastomères de silicone polyglycérolés.

L'élastomère de silicone polyglycérolé est un organopolysiloxane réticulé élastomère pouvant être obtenu par réaction d'addition réticulation de diorganopolysiloxane contenant au moins un hydrogène lié au silicium et de composés polyglycérolés ayant des groupements à insaturation éthylénique, notamment en présence de catalyseur platine.

De préférence, l'organopolysiloxane réticulé élastomère est obtenu par réaction d'addition réticulation (A) de diorganopolysiloxane contenant au moins deux hydrogènes liés chacun à un silicium, et (B) de composés glycérolés ayant au moins deux groupements à insaturation éthylénique, notamment en présence (C) de catalyseur platine.

En particulier, l'organopolysiloxane peut être obtenu par réaction de composé polyglycérolé à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles ayant de 1 à 18 atomes de carbone, tels que méthyle, éthyle, propyle, butyle, octyle, décyle, dodécyle (ou lauryle), myristyle, cétyle, stéaryle ; des groupes alkyles substitués tels que 2-phényléthyle, 2-phénylpropyle, 3,3,3-trifluoropropyle ; des groupes aryles tels que phényle, tolyle, xylyle ; des groupes aryles substitués tels que phényléthyle ; et des groupes hydrocarbonés monovalents substitués tels qu'un groupe époxy, un groupe ester carboxylate, ou un groupe mercapto. De préférence, ledit groupe organique est choisi parmi les groupes méthyle, phényle, lauryle.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (B) peut être un composé polyglycérolé répondant à la formule (B') suivante :

CₘH₂ₘ₋₁-O-[ Gly ]ₙ-CₘH₂ₘ₋₁ (B')

dans laquelle m est un entier allant de 2 à 6, n est un entier allant de 2 à 200, de préférence allant de 2 à 100, de préférence allant de 2 à 50, de préférence n allant de 2 à 20, de préférence allant de 2 à 10, et préférentiellement allant de 2 à 5, et en particulier égal à 3 ; Gly désigne :

-CH₂-CH(OH)-CH₂-O- ou -CH₂-CH(CH₂OH)-O-

Avantageusement, la somme du nombre de groupements éthyléniques par molécule du composé (B) et du nombre d'atomes d'hydrogène liés à des atomes de silicium par molécule du composé (A) est d'au moins 4.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1/1 à 20/1.

Le composé (C) est le catalyseur de la réaction de réticulation, et est notamment l'acide chloroplatinique, les complexes acide chloroplatinique-oléfine, les complexes acide chloroplatinique-alkénylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

L'élastomère de silicone polyglycérolé selon l'invention est généralement mélangé avec au moins une huile hydrocarbonée et/ou une huile siliconée pour former un gel. Dans ces gels, l'élastomère polyglycérolé est souvent sous forme de particules non-sphériques.

De tels élastomères sont notamment décrits dans la demande de brevet WO 2004/024798.

Comme élastomères de silicone polyglycérolés, on peut utiliser ceux vendus sous les dénominations "KSG-710", "KSG-810", "KSG-820", "KSG-830", "KSG-840" par la société Shin Etsu.

Selon un mode préféré, on utilise un élastomère de silicone émulsionnant polyglycérolé à 25 % en poids dans du polydimethylsiloxane (6 cSt), notamment commercialisé sous la dénomination KSG-710 par la société Shin Etsu.

Selon un aspect de l'invention, ces élastomères de silicone peuvent être présents dans la composition selon l'invention en une teneur totale en matière active au moins supérieure à 0,8 % en poids, par rapport au poids total de la composition, notamment allant de 1 à 4 % en poids, de préférence supérieure ou égale à 1,5 % en poids, notamment allant de 1,5 à 2,5 % en poids, et plus préférentiellement supérieure ou égale à 1,6 % en poids, notamment allant de 1,6 à 2 % en poids.

Une composition selon l'invention comprend donc comme architecture de base, une phase aqueuse dispersée dans une phase huileuse et au moins un tensioactif choisi parmi les tensioactifs siliconés ou hydrocarbonés.
En fonction de la viscosité de la composition recherchée pour être dans le profil rhéologique selon l'invention, l'homme du métier pourra ajuster les teneurs en phase aqueuse, phase huileuse et/ou en tensioactifs.

Il pourra également avantageusement intégrer dans cette architecture de base un ou plusieurs ingrédients destinés à moduler, en particulier augmenter la viscosité de ladite composition. En particulier, la composition selon l'invention comprend au moins un agent augmentant la viscosité de ladite composition.
Comme agents susceptibles d'augmenter la viscosité d'une composition, on peut citer notamment, les gélifiants lipophiles minéraux ou organiques (notamment polymériques), les stucturants lipophiles, les matériaux particulaires choisis parmi les charges, les pigments, les nacres, et leurs mélanges. Ces ingrédients additionnels pourront apporter en outre des propriétés cosmétiques de texture, de confort, de tenue et éventuellement de couleur, recherchées dans le domaine du soin et/ou du maquillage.
Ainsi une composition de l'invention pourra comprendre avantageusement au moins un agent choisi parmi les gélifiants lipophiles minéraux ou organiques (notamment polymériques), les stucturants lipophiles, les matériaux particulaires choisis parmi les charges, les pigments, les nacres, et leurs mélanges.

Selon un mode particulier de l'invention, la composition comprend au moins un gélifiant lipophile.

### AGENTS GELIFIANTS LIPOPHILES

Un agent gélifiant convenant à l'invention est avantageusement lipophile. Un gélifiant lipophile peut être minéral ou organique (notamment polymérique).

Le ou les gélifiants lipophiles pourront être présents dans la composition en une teneur allant de 0,1 à 20% en poids, en particulier de 0,2 à 15% en poids, et mieux de 0,25 à 10% en poids par rapport au poids total de ladite composition.

### Gélifiants minéraux

Comme gélifiants lipophiles, on peut citer par exemple les argiles modifiées hydrophobes telles que le silicate de magnésium modifié (Bentone gel VS38 de RHEOX), l'hectorite modifiée par le chlorure de distéaryl diméthyl ammonium (nom CTFA : Disteardimonium hectorite) commercialisé sous la dénomination « Bentone 38 CE » par la société RHEOX.

Comme gélifiant lipophile minéral, on peut citer les argiles éventuellement modifiées comme les hectorites modifiées par un chlorure d'ammonium d'acide gras en C₁₀ à C₂₂, comme l'hectorite modifiée par du chlorure de di-stéaryl di-méthyl ammonium telle que, par exemple, celle commercialisée sous la dénomination de Bentone 38V^{®} par la société ELEMENTIS.

### Gélifiants organiques polymériques

Les gélifiants lipophiles organiques polymériques sont par exemple les organopolysiloxanes élastomériques partiellement ou totalement réticulés, de structure tridimensionnelle, comme ceux commercialisés sous les dénominations de KSG6^{®}, KSG16^{®} et de KSG18^{®} par la société SHIN-ETSU, de Trefil E-505C^{®} et Trefil E-506C^{®} par la société DOW-CORNING, de Gransil SR-CYC^{®}, SR DMF10^{®}, SR-DC556^{®}, SR 5CYC gel^{®}, SR DMF 10 gel^{®} et de SR DC 556 gel^{®} par la société GRANT INDUSTRIES, de SF 1204^{®} et de JK 113^{®} par la société GENERAL ELECTRIC ; l'éthylcellulose comme celle vendue sous la dénomination Ethocel^{®} par la société DOW CHEMICAL ; les polycondensats de type polyamide résultant de la condensation entre un acide dicarboxylique comprenant au moins 32 atomes de carbone et un alkylène diamine et en particulier l'éthylène diamine, dans lequel le polymère comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG^{®} par la société ARIZONA CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en C₁ à C₆, et en particulier en C₁ à C₃ et leurs mélanges.

Comme agents gélifiants lipophiles convenant à l'invention peuvent également être cités les copolymères du type polystyrène/polyalkylène, et plus particulièrement les copolymères séquencés de type « dibloc », « tribloc » ou « radial » du type polystyrène/polyisoprène, polystyrène/polybutadiène tels que ceux commercialisés sous la dénomination Luvitol HSB^{®} par la société BASF, du type polystyrène/copoly(éthylène-propylène) tels que ceux commercialisés sous la dénomination de Kraton^{®} par la société KRATON POLYMERS ou encore du type polystyrène/copoly(éthylène-butylène), les mélanges de copolymères tribloc et radial (en étoile) dans l'isododécane tels que ceux commercialisé par la société PENRECO sous la dénomination Versagel^{®} comme par exemple le mélange de copolymère tribloc butylène/éthylène/styrène et de copolymère étoile éthylène/propylène/styrène dans l'isododécane (Versagel M 5960).

Parmi les gélifiants lipophiles pouvant être utilisés dans une composition cosmétique de l'invention, on peut encore citer les esters de dextrine et d'acide gras, tels que les palmitates de dextrine, notamment tels que ceux commercialisés sous les dénominations Rheopearl TL^{®} ou Rheopearl KL^{®} par la société CHIBA FLOUR, les huiles végétales hydrogénées, telles que l'huile de ricin hydrogénée, les alcools gras, en particulier de C₈ à C₂₆, et plus particulièrement de C₁₂ à C₂₂, comme par exemple, l'alcool mysritylique, l'alcool cétylique, l'alcool stéarylique ou l'alcool béhénylique.

Selon un mode de réalisation particulièrement préféré, une composition selon l'invention peut comprendre au moins un agent gélifiant lipophile minéral choisi parmi les argiles modifiées hydrophobes.

Selon un mode de réalisation particulièrement préféré, une composition selon l'invention peut comprendre au moins un agent gélifiant lipophile organique polymérique, en particulier choisi parmi les copolymères du type polystyrène/polyalkylène, et plus particulièrement les copolymères du type polystyrène/copoly(éthylène-propylène), notamment ceux commercialisés sous la dénomination Kraton^{®} par la société KRATON POLYMERS.

### AGENTS STRUCTURANTS LIPOPHILES

La phase grasse peut également comprendre au moins un agent strucurant lipophile.

Le ou les agents structurants lipophiles pourront être présents dans la composition en une teneur allant de 0,05 à 10% en poids, en particulier de 0,1 à 8% en poids, et mieux de 0,2 à 5% en poids par rapport au poids total de ladite composition.

En particulier, ledit agent structurant lipophile peut être choisi parmi au moins une cire, au moins une gomme et/ou au moins un corps gras pâteux, d'origine végétale, animale, minérale ou de synthèse, voire siliconé, et leurs mélanges.

### Cires

Parmi les cires solides à température ambiante, susceptibles d'être présentes dans la composition selon l'invention, on peut citer les cires hydrocarbonées telles que la cire d'abeilles, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch, les huiles hydrogénées, les esters gras et les glycérides concrets à 25 °C. On peut également utiliser des cires de silicone, parmi lesquelles on peut citer les alkyl, alcoxy et/ou esters de polyméthylsiloxane. Les cires peuvent se présenter sous forme de dispersions stables de particules colloïdales de cire telles qu'elles peuvent être préparées selon des méthodes connues, telles que celles de "Microemulsions Theory and Practice", L. M. Prince Ed., Academic Press (1977), pages 21-32. Comme cire liquide à température ambiante, on peut citer l'huile de Jojoba.

On peut également mentionner les huiles végétales hydrogénées, telles que l'huile de ricin hydrogénée.

A titre de structurant lipophile convenant également à l'invention, on peut mentionner les alcools gras, en particulier de C₈ à C₂₆, et plus particulièrement de C₁₂ à C₂₂.

Selon un mode de réalisation, un alcool gras convenant à l'invention peut être choisi parmi l'alcool mysritylique, l'alcool cétylique, l'alcool stéarylique, l'alcool béhénylique.

A titre de structurant lipophile convenant également à l'invention, on peut mentionner les esters d'acide gras et de glycérols, tels que le tristéarate de glycéryle.

Les cires peuvent être présentes à raison de 0,1 % à 10 %, en poids, par rapport au poids total de l'émulsion, et de préférence de 0,1 % à 5 % en poids.

### Composés gras pâteux

On peut définir les composés gras pâteux à l'aide d'au moins une des propriétés physico-chimiques suivantes :
- une viscosité de 0,1 à 40 Pa.s (1 à 400 poises), de préférence 0,5 à 25 Pa.s, mesurée à 40 °C avec un viscosimètre rotatif CONTRAVES TV équipé d'un mobile MS-r3 ou MS-r4 à la fréquence de 60 Hz,
- un point de fusion de 25-70 °C, de préférence 25-55 °C.

Les compositions de l'invention peuvent également comprendre au moins une alkyl-, alkoxy- ou phényl-diméthicone telle que, par exemple le produit vendu sous la dénomination de "Abil wax 2440" par la Société GOLDSCHMIDT.

La composition selon l'invention peut comprende un ou plusieurs matériau(x) particulaire(s) choisis parmi les charges, les matières colorantes pulvérulentes et leurs mélanges, constituant la phase pulvérulente de ladite composition.

### PHASE PULVERULENTE

Au sens de la présente invention, "phase pulvérulente" couvre toutes particules de type matières colorantes et/ou charges, telles que définis ci-après.

En particulier, la composition comprend au moins un matériau particulaire choisi parmi les pigments, les charges et leurs mélanges, en particulier les pigments

Selon un mode de réalisation particulier, une composition selon l'invention peut comprendre au moins 3 % en poids, notamment plus de 10 %, voire plus de 12 % en poids d'une phase pulvérulente. Celle-ci y est dispersée sous une forme homogène et stabilisée.

Selon un mode de réalisation, la composition de l'invention comprend au moins une charge.

### CHARGES

Une composition conforme à l'invention peut également comprendre au moins une charge, de nature organique ou minérale.

Par « charge », il faut comprendre les particules incolores ou blanches, solides de toutes formes, qui se présentent sous une forme insoluble et dispersée dans le milieu de la composition. De nature minérale ou organique, elles permettent de conférer à la composition de la douceur, de la matité et de l'uniformité au maquillage.

Les charges peuvent être présentes dans l'émulsion en une teneur allant de 0,1 % à 10 % en poids, par rapport au poids total de l'émulsion, de préférence 0,5 % à 7 %.

Les charges utilisées dans les compositions selon la présente invention peuvent être de formes lamellaires, globulaires, sphériques, de fibres ou de toute autre forme intermédiaire entre ces formes définies.

Les charges selon l'invention peuvent être ou non enrobées superficiellement, et, en particulier, elles peuvent être traitées en surface par des silicones, des acides aminés, des dérivés fluorés ou toute autre substance favorisant la dispersion et la compatibilité de la charge dans la composition.

Parmi les charges minérales utilisables dans les compositions selon l'invention, on peut citer le talc, le mica, la silice, le siloxysilicate de triméthyle, le kaolin, la bentone, le carbonate de calcium et l'hydrogéno-carbonate de magnésium, l'hydroxyapatite, le nitrure de bore, les microsphères de silice creuses (Silice Beads de Maprecos), les microcapsules de verre ou de céramique, les charges à base de silice comme l'Aerosil 200, l'Aerosil 300 ; la Sunsphere H-33, la Sunsphere H-51 commercialisés par Asahi Glass ; le Chemicelen commercialisé par Asahi Chemical ; les composites de silice et de dioxyde de titane, comme la série TSG commercialisée par Nippon Sheet Glass, et leurs mélanges.

Parmi les charges organiques utilisables dans les compositions selon l'invention on peut citer les poudres de polyamide (Nylon^{®} Orgasol de chez Atochem), de poly-b-alanine et polyéthylène, les poudres de polytétrafluoroéthylène (Téflon^{®}), la lauroyl-lysine, l'amidon, les poudres de polymères de tétrafluoroéthylène, les microsphères creuses de polymères, telles l'EXPANCEL (NOBEL INDUSTRIE), les savons métalliques dérivés d'acides organiques carboxyliques ayant de 8 à 22 atomes de carbone, de préférence, de 12 à 18 atomes de carbone, par exemple, le stéarate de zinc, de magnésium ou de lithium, le laurate de zinc, le myristate de magnésium, le Polypore^{®} L 200 (Chemdal Corporation), les microbilles de résine de silicone (Tospearl^{®} de Toshiba, par exemple), les poudres de polyuréthane, en particulier, les poudres de polyuréthane réticulé comprenant un copolymère, ledit copolymère comprenant du triméthylol hexyllactone, comme le polymère d'hexaméthylène di-isocyanate/triméthylol hexyllactone, commercialisé sous la dénomination de PLASTIC POWDER D-400^{®} ou PLASTIC POWDER D-800^{®} par la société TOSHIKI, les microcires de Carnauba, telles que celle commercialisée sous la dénomination de MicroCare 350^{®} par la société MICRO POWDERS, les microcires de cire synthétique, telles que celle commercialisée sous la dénomination de MicroEase 114S^{®} par la société MICRO POWDERS, les microcires constituées d'un mélange de cire de Carnauba et de cire de polyéthylène, telles que celles commercialisées sous les dénominations de Micro Care 300^{®} et 310^{®} par la société MICRO POWDERS, les microcires constituées d'un mélange de cire de Carnauba et de cire synthétique, telles que celle commercialisée sous la dénomination Micro Care 325^{®} par la société MICRO POWDERS, les microcires de polyéthylène, telles que celles commercialisées sous les dénominations de Micropoly 200^{®}, 220^{®}, 220L^{®} et 250S^{®} par la société MICRO POWDERS ;les fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser. les fibres ont une longueur allant de 1 µm à 10 mm, de préférence de 0,1 mm à 5 mm et mieux de 0,3 mm à 3 mm. Leur section peut être comprise dans un cercle de diamètre allant de 2 nm à 500 µm, de préférence allant de 100 nm à 100 µm et mieux de 1 µm à 50 µm. A titre de fibres utilisables dans les compositions selon l'invention, on peut citer les fibres non rigides telles que les fibres de polyamide (Nylon^{®}) ou les fibres rigides telles que les fibres de polyimide-amide comme celles vendues sous les dénominations KERMEL^{®}, KERMEL TECH^{®} par la société RHODIA ou de poly-(p-phénylène-téréphtalamide) (ou d'aramide) notamment vendues sous la dénomination Kevlar ^{®} par la société DUPONT DE NEMOURS,
et leurs mélanges.

Selon un mode de réalisation, la composition de l'invention comprend au moins une matière colorante pulvérulente, en particulier au moins un pigment.

### MATIERES COLORANTES PULVERULENTES

Une composition selon l'invention peut en outre comprendre au moins une matière colorante pulvérulente.

Les matières colorantes pulvérulentes peuvent être présentes à raison de 0,01 à 30 % en poids, notamment, de 0,1 à 20 % en poids, et en particulier, de 1 à 15 % en poids, par rapport au poids total de la composition cosmétique.

Une composition cosmétique conforme à l'invention peut, avantageusement, incorporer au moins une matière colorante choisie parmi des matières colorantes pulvérulentes organiques ou inorganiques, notamment de type pigments ou nacres classiquement utilisés dans les compositions cosmétiques, des matériaux à effet optique spécifique, et leurs mélanges.

### Pigments

Par pigments, il faut comprendre des particules blanches ou colorées, minérales ou organiques, insolubles dans une solution aqueuse, destinées à colorer et/ou opacifier le film résultant.

Comme pigments minéraux utilisables dans l'invention, on peut citer les oxydes de titane, de zirconium ou de cérium, ainsi que les oxydes de zinc, de fer ou de chrome, le bleu ferrique, le violet de manganèse, le bleu outremer et l'hydrate de chrome.

On utilise de préférence des pigments d'oxydes de fer ou de dioxyde de titane.

Il peut également s'agir de pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

La matière colorante peut encore comporter un pigment ayant une structure qui peut être, par exemple, de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Avantageusement, les pigments peuvent être présents sous une forme enrobée hydrophobe dans l'émulsion selon l'invention. Il s'agit plus particulièrement de pigments traités en surface avec un agent hydrophobe pour les rendre compatibles avec la phase grasse de l'émulsion, notamment pour qu'ils aient une bonne mouillabilité avec les huiles de la phase grasse. Ainsi, ces pigments traités sont bien dispersés dans la phase grasse.

L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zinc, de sodium, de potassium. L'acide aminé peut être par exemple la lysine, l'acide glutamique, l'alanine.

Le terme alkyle mentionné dans les composés cités précédemment désigne notamment un groupe alkyle ayant de 1 à 30 atomes de carbone, de préférence ayant de 5 à 16 atomes de carbone.

Des pigments traités hydrophobes sont notamment décrits dans la demande EP-A-1086683.

Les pigments peuvent être présents en une teneur allant de 0,01 % à 20 % en poids, par rapport au poids total de la composition, de préférence en une teneur au moins égale à 5 % en poids, notamment allant de 5 % à 20 % en poids, plus particulièrement de 8 % à 20 % en poids, et préférentiellement allant de 8 à 15 % en poids.

### Nacres

Par « nacres », il faut comprendre des particules colorées de toute forme, irisées ou non, notamment, produites par certains mollusques dans leur coquille ou bien synthétisées et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

### Matériaux à effet optique

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques, comme, par exemple, les pigments monochromatiques. Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment, interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :
- les particules d'au moins un métal et/ou d'au moins un dérivé métallique,
- les particules comportant un substrat organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple, les bronzes et les laitons) sont des métaux préférés.

Par « dérivés métalliques », on désigne des composés dérivés de métaux, notamment, des oxydes, des fluorures, des chlorures et des sulfures

L'agent de coloration goniochromatique peut être choisi, par exemple, parmi les structures multicouches interférentielles et les agents de coloration à cristaux liquides.

### Adjuvants cosmétiques

Les compositions de l'invention peuvent contenir en outre un ou plusieurs des adjuvants habituels dans les domaines cosmétique et dermatologique, tels que des agents hydratants ; des émollients ; des actifs hydrophiles ou lipophiles ; des agents anti-radicaux libres ; des séquestrants ; des antioxydants ; des conservateurs ; des agents alcanisants ou acidifiants ; des parfums ; des gélifiants hydrophiles ; des agents filmogènes, en particulier des polymères filmogènes (pour des compositions dans un axe tenue) ; des filtres solaires organiques ou physiques, des colorants hydrosolubles ou liposolubles; et leurs mélanges. Les quantités de ces différents adjuvants sont celles classiquement utilisées dans les fonds de teint.

Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.

Ces adjuvants sont généralement présents dans la composition en une teneur allant de 0,01 à 20 % en poids, de préférence de 0,1 à 10 % en poids par rapport au poids total de ladite composition.

L'invention est illustrée dans les exemples présentés ci-après à titre illustratif et non limitatif du domaine de l'invention :
Sauf indication contraire, les valeurs dans les exemples ci-dessous sont exprimées en % en poids par rapport au poids total de la composition.

### EXEMPLES

### Exemples 1 et 2 : Fond de teint : Influence de la viscosité (E/H à phase huileuse siliconée)

| | | Exemple 1 (invention) | Exemple 2 (comparatif) |
|---|---|---|---|
| | | % massique | % massique |
| A | Dimethicone copolyol vendu sous la référence KF 6017 par la société Shin Etsu | 1,4881 | 1,9981 |
| | Bis PEG/PPG-14/14 dimethicone + Cyclopentasiloxane vendu sous la référence ABIL EM 97 par la société Goldschmidt | 0,66 | 1,00 |
| | Cyclopentasiloxane | 10,9084 | 17,6484 |
| | Phenyl trimethicone vendu sous la référence DC556 par la société Dow Corning | 1,31 | 2,00 |
| | Ethyl hexyl methoxycinnamate | 1,97 | 3,00 |
| | Squalane | 0,66 | 1,00 |
| B | Cyclopentasiloxane | 7,00 | 7,00 |
| | Oxyde de fer jaune enrobé de stearoyl glutamate d'aluminium (3%) NAI-C33-9001-10 de la société Miyoshi Kasei | 1,1715 | 1,1715 |
| | Oxyde de fer rouge enrobé de stearoyl glutamate d'aluminium (3%) NAI-C33-8001-10 de la société Miyoshi Kasei | 0,3430 | 0,3430 |
| | Oxyde de fer noir enrobé de stearoyl glutamate d'aluminium (3%) NAI-C33-7001-10 de la société Miyoshi Kasei | 0,108 | 0,108 |
| | Dioxyde de titane (anatase) enrobé de stearoyl glutamate d'aluminium (97/3) NAI-TAO-77891 de la société Miyoshi Kasei | 10,381 | 10,381 |
| C | Talc vendu sous la référence Micro Ace P3 par la société Nippon Talc | 0,33 | 0,50 |
| | Poudre de nylon 12 vendue sous la référence SP 500 par la société Toray | 0,33 | 0,50 |
| D | Eau déminéralisée | 46,14 | 36,15 |
| | Butylene glycol | 3,00 | 3,00 |
| | Sulfate de magnésium | 0,70 | 0,70 |
| | Maltitol + Sorbitol vendu sous la référence Mabit par la société Hayashibara | 0,50 | 0,50 |
| E | Ethanol | 13,00 | 13,00 |
| | TOTAL | 100% | 100% |

### Mode opératoire

On pèse les constituants de la phase A dans le bêcher principal et l'on agite en maintenant à température ambiante sous Moritz (1000 tr/min).
La phase B est préparée séparément en broyant trois fois à la tricylindre, le mélange de pigments et de cyclopentasiloxane.
Cette phase B est ensuite ajoutée en maintenant l'agitation, ainsi que les charges (phase C).

La phase aqueuse D est aussi préparée séparément, en pesant dans un bêcher le butylène glycol, le sulfate de magnésium, le maltitol + sorbitol et l'eau à température ambiante.
La phase aqueuse D est placée sous agitation à l'aide d'un barreau magnétique jusqu'à homogénéisation.
L'émulsion se fait à température ambiante : on verse la phase aqueuse D dans la phase grasse en augmentant progressivement la vitesse d'agitation (Moritz) jusqu'à 4000 tours/mn. On maintient l'agitation pendant 7 mn.
Puis on ajoute enfin la phase E (éthanol) pendant les 3 dernières minutes de l'émulsification.

### Résultats de viscosité

| **Viscosité (Pa s)** | **Exemple 1 (Invention)** | **Exemple 2 (comparatif)** |
|---|---|---|
| Bas gradient 0,01s⁻¹ | 105 | 66,4 |
| Haut gradient 1000 s⁻¹ | 0,28 | 0,18 |

### Evaluation sensorielle

Dans le cas de l'exemple 1 (invention), l'applicateur tel que décrit dans les figures 1 et 2 (orifice de 3 mm) est parfaitement amorcé : la pression à exercer sur le réservoir de l'applicateur est faible et la quantité de fond de teint délivré sur le rouleau est contrôlée. La quantité de fond de teint, restitué par le rouleau lors de son application sur la peau, est adaptée et le résultat maquillage est homogène.
En revanche, dans le cas de l'exemple 2 (comparatif), malgré un amorçage facile de l'applicateur, la quantité de fond de teint restitué sur le rouleau est trop importante entrainant des coulures sur la tête de l'applicateur. Il s'ensuit un résultat maquillage moins homogène que dans le cas de l'exemple 1.

### Exemple 3 : Fond de teint (E/H à phase huileuse hydrocarbonée)

| | | % massique |
|---|---|---|
| A1 | PEG-30 Dipolyhydroxy stearate vendu sous la référence ARLACEL P 135 par la Société UNIQEMA | 2,98 |
| | Tristéarine + Stéarate d'ethylene glycol acétylé vendu sous la référence UNITWIX par la société UNITED GUARDIAN | 0,52 |
| | Dicapryl carbonate | 2,24 |
| | Hexacaprylate/caprate de dipentaérythrityle vendu sous la référence DUB DPHCC par la société STEARINERIE DUBOIS | 7,46 |
| | Ethyl hexyl methoxycinnamate | 5,22 |
| | Néopentanoate d'isodécyle | 4,55 |
| | Gel de bentone vendu sous la référence BENTONE GEL IHD V par la société ELEMENTIS | 3,84 |
| A2 | Isohexadecane | 5,63 |
| | Oxyde de fer jaune enrobé de stearoyl glutamate d'aluminium (3%) NAI-C33-9001-10 de la société Miyoshi Kasei | 1,6 |
| | Oxyde de fer rouge enrobé de stearoyl glutamate d'aluminium (3%) NAI-C33-8001-10 de la société Miyoshi Kasei | 0,45 |
| | Oxyde de fer noir enrobé de stearoyl glutamate d'aluminium (3%) NAI-C33-7001-10 de la société Miyoshi Kasei | 0,11 |
| | Dioxyde de titane (anatase) enrobé de stearoyl glutamate d'aluminium (97/3) NAI-TAO-77891 de la société Miyoshi Kasei | 9,84 |
| A3 | Copolymère Methyl methacrylate/Ethylene glycol di methacrylate vendu sous la référence TECHPOLYMER MBP-8 par la société SEKISUI PLASTICS | 3,00 |
| A4 | Acétate de tocophérol | 0,075 |
| B | Eau déminéralisée | 46,485 |
| | Glycérol | 5,00 |
| | Methyl paraben | 0,3 |
| | Sulfate de magnésium | 0,60 |
| | EDTA | 0,10 |
| | TOTAL | 100% |

### Mode opératoire

On pèse les constituants de la phase A1, à l'exception du gel de bentone, dans le bêcher principal et on place le bêcher au bain-marie jusqu'à la fusion de la cire.
On ajoute ensuite, à température ambiante, le gel de bentone et l'on agite à l'aide d'un agitateur Moritz (1000 tr/min).
La phase A2 est préparée séparément en broyant trois fois à la tricylindre, le mélange de pigments et d'isohexadécane.
Cette phase A2 est ensuite ajoutée à température ambiante en maintenant l'agitation, ainsi que les phases A3 et A4.
La phase aqueuse B est aussi préparée séparément, en pesant dans un bêcher le glycérol, le sulfate de magnésium, l'EDTA, le méthyl parabène et en ajoutant l'eau préalablement chauffée à 95°C.
La phase aqueuse B est placée sous agitation à l'aide d'un barreau magnétique jusqu'à homogénéisation.
L'émulsion se fait à température ambiante : on verse la phase aqueuse B dans la phase grasse en augmentant progressivement la vitesse d'agitation (Moritz) jusqu'à 4000 tours/mn. On maintient l'agitation pendant 10 mn.

### Résultats de viscosité

| **Viscosité (Pa s)** | **Exemple 3 (Invention)** |
|---|---|
| Bas gradient 0,01s⁻¹ | 368 |
| Haut gradient 1000 s⁻¹ | 0,45 |

### Evaluation sensorielle

Comme dans le cas de l'exemple 1, l'amorçage de l'applicateur tel que décrit dans les figures 1 et 2 (orifice 3 mm) est très satisfaisant : la pression à exercer sur le réservoir de l'applicateur est faible et la quantité de fond de teint délivré sur le rouleau est contrôlée. De plus, la quantité de fond de teint, restitué par le rouleau lors de son application sur la peau, est adaptée et le résultat maquillage est homogène.

Les exemples 1 (E/H à phase huileuse siliconée) et 3 (E/H à phase huileuse hydrocarbonée) ensemble, montrent que la nature des huiles dans la phase huileuse, siliconée ou hydrocarbonée, n'a pas ou peu d'impact sur le résultat : c'est le profil rhéologique de la composition qui a son importance.

## Revendications

1. Ensemble de conditionnement et d'application cosmétique de maquillage et/ou de soin des matières kératiniques comprenant au moins :
- un récipient contenant au moins une composition cosmétique, sous forme d'émulsion eau-dans-huile **caractérisée par** le profil rhéologique suivant :
- une viscosité comprise entre 35 et 1000 Pa s, en particulier entre 40 et 950 Pa s, de préférence entre 55 et 900 Pa s, à un gradient de cisaillement de 0,01 s⁻¹,
- une viscosité comprise entre 0,20 et 0,90 Pa s, en particulier entre 0,25 et 0,80 Pa s, de préférence entre 0,28 et 0,70 Pa s, à un gradient de cisaillement de 1000 s⁻¹,
telles que mesurées à l'aide d'un rhéomètre à contrainte imposée, ARG2 TA instrument, à 25°C, et
- une tête applicatrice (3) solidaire dudit récipient et supportant un organe d'application (7), ledit organe d'application comportant une surface d'application apte, en réponse à son déplacement en engagement avec les matières kératiniques, à tourner autour d'au moins un axe ou d'un centre de rotation, ladite surface d'application étant en communication fluidique avec ledit récipient via au moins un passage qui, sur au moins une partie de sa longueur, présente un diamètre de 1 à 6mm, de préférence de 2 à 5mm, et mieux de 2 à 4mm, ledit organe d'application étant sous la forme d'un rouleau.

2. Ensemble de conditionnement et d'application cosmétique selon la revendication 1, dans lequel le passage débouche en regard de ladite surface d'application via au moins un orifice présentant un diamètre de 1 à 6mm, de préférence de 2 à 5mm, et mieux de 2 à 4mm.

3. Ensemble de conditionnement et d'application cosmétique selon la revendication 1 ou 2, dans lequel ladite composition comprend de 10% à 80% en poids de phase aqueuse, par rapport au poids total de ladite composition.

4. Ensemble de conditionnement et d'application cosmétique selon l'une des revendications 1 à 3, dans lequel ladite composition comprend de 10% à 90% en poids de phase grasse liquide, par rapport au poids total de ladite composition.

5. Ensemble de conditionnement et d'application cosmétique selon la revendication précédente, dans lequel la phase grasse liquide comprend des huiles choisies parmi les huiles volatiles ou non volatiles, de type hydrocarbonées ou siliconées, et leurs mélanges.

6. Ensemble de conditionnement et d'application cosmétique selon l'une quelconque des revendications précédentes, dans lequel ladite composition comprend au moins un tensioactif, notamment choisi parmi les tensioactifs amphotères, anioniques, cationiques ou non ioniques, utilisés seuls ou en mélange.

7. Ensemble de conditionnement et d'application cosmétique selon la revendication précédente, dans lequel le(s) tensioactif(s) est/sont présent(s) en une teneur totale allant de 0,5% à 15% en poids, par rapport au poids total de la composition.

8. Ensemble de conditionnement et d'application cosmétique selon l'une quelconque des revendications précédentes, dans lequel la composition comprend en outre au moins un agent choisi parmi les agents gélifiants lipophiles minéraux ou organiques, les structurants lipophiles, les matériaux particulaires choisis parmi les charges, les matières colorantes pulvérulentes, et leurs mélanges.

9. Ensemble de conditionnement et d'application cosmétique selon l'une quelconque des revendications précédentes, dans lequel la composition comprend au moins un matériau particulaire choisi parmi les pigments, les charges et leurs mélanges, en particulier les pigments.

10. Ensemble de conditionnement et d'application cosmétique selon l'une quelconque des revendications précédentes, la composition constituant un fard à joues, un fard à paupières, un fond de teint, un produit anticerne, un rouge à lèvres, un produit de maquillage du corps, un produit de soin pour le visage, un produit de soin du corps ou un produit antisolaire.

11. Ensemble de conditionnement et d'application cosmétique selon l'une quelconque des revendications précédentes, la composition constituant un fond de teint.

12. Ensemble de conditionnement et d'application cosmétique selon la revendication 1, dans lequel l'organe d'application sous forme d'un rouleau possède une surface d'application de type mousse à cellules ouvertes ou fermées, éventuellement floquées, flocage, élastomère, matériau fritté, matériau tissé ou non tissé.

13. Procédé de maquillage et/ou de soin d'une matière kératinique, en particulier la peau, dans lequel :
on prélève et on applique simultanément une quantité d'une composition, à l'aide d'un dispositif d'application comprenant un récipient contenant ladite composition et une tête applicatrice solidaire dudit récipient supportant un organe d'application sous la forme d'un rouleau, ledit organe d'application comportant une surface d'application apte, en réponse à son déplacement en engagement avec les matières kératiniques, à tourner autour d'un axe ou d'un centre de rotation, ladite composition étant sous forme d'émulsion eau-dans-huile **caractérisée par** le profil rhéologique suivant :
- une viscosité comprise entre 35 et 1000 Pa s, en particulier entre 40 et 950 Pa s, de préférence entre 55 et 900 Pa s, à un gradient de cisaillement de 0,01 s⁻¹,
- une viscosité comprise entre 0,20 et 0,90 Pa s, en particulier entre 0,25 et 0,80 Pa s, de préférence entre 0,28 et 0,70 Pa s, à un gradient de cisaillement de 1000 s⁻¹, telles que mesurées à l'aide d'un rhéomètre à contrainte imposée, ARG2 TA instrument, à 25°C,
ladite surface d'application étant en communication fluidique avec ledit récipient via au moins un passage qui, sur au moins une partie de sa longueur, présente un diamètre de 1 à 6mm, de préférence de 2 à 5mm, et mieux de 2 à 4mm.

14. Procédé selon la revendication précédente, dans lequel ladite composition est telle que définie selon l'une quelconque des revendications 3 à 11.
